# EUROPÄISCHE PATENTANMELDUNG

(11) **EP 0 826 656 A1**
(43) Veröffentlichungstag der Anmeldung: **04.03.1998**
(21) Anmeldenummer: 97114737.6
(22) Anmeldetag: 26.08.1997
(51) Int. Cl.: C07C 17/08

(54) **Verfahren zur Herstellung von Alkylbromiden aus wässriger Bromwasserstoffsäure und Olefinen**

(30) Priorität: 28.08.1996 DE 19634818
(71) Anmelder: BASF AKTIENGESELLSCHAFT, 67056 Ludwigshafen (DE)
(72) Erfinder: Eiermann, Matthias, Dr., 67117 Limburgerhof (DE); Ebel, Klaus, Dr., 68623 Lampertheim (DE)
(74) Vertreter: Isenbruck, Günter, Dr.

(57) **Zusammenfassung**

In dem Verfahren zur Herstellung von Alkylbromiden aus Mischungen, enthaltend wäßrige Bromwasserstoffsäure und Olefine, wird während der Umsetzung die vorhandene wäßrige Bromwasserstoffsäure aufkonzentriert.

## Beschreibung

Alkylbromide haben in der Organischen Chemie eine große Bedeutung für eine Vielzahl von Anwendungen, beispielsweise für metallorganische Reaktionen. Allgemein bekannt ist, sie aus den entsprechenden Alkoholen durch Umsetzung mit Thionylbromid oder Bromwasserstoffsäure herzustellen. Auch durch Addition von HBr an Olefine sind Alkylbromide zugänglich; dabei wird Bromwasserstoffgas in einem nichtwäßrigen Medium wie beispielsweise Eisessig eingesetzt. Als Katalysatoren bei der Umsetzung des Gases dienen auch Lewissäuren wie Eisen(III)salze um die Additionsreaktion zu beschleunigen (vgl. Houben-Weyl. Bd. V/4). Bromwasserstoffgas ist jedoch teuer und in technischem Maßstab nicht beliebig verfügbar.

Aus J. Am. Chem. Soc. 1934, 56, 926 (M. L. Sherrill, K. E. Mayer, G. F. Walker) ist bekannt, daß die Addition von Bromwasserstoffsäure in konzentrierter wäßriger Lösung an Olefine wie 1-Penten oder 1-Hepten sehr langsam verläuft. Die Reaktion bedarf großer Überschüsse an Bromwasserstoffsäure, so daß dieser Synthese keine wirtschaftliche Bedeutung zukommt.

Durch Zusatz von Oniumsalzen (D. Landini et al. J. Org. Chem. 1980, 45, 3527), von Radikalinitiatoren (vgl. NL-A 65 03537) oder von stöchiometrischen Mengen Phosphortribromid (SU-A 825 477) kann die Reaktionsgeschwindigkeit verbessert und die Ausbeute erhöht werden. Jedoch sind die benötigten Reagenzien teuer und führen auch im Falle der Radikalinitiatoren bei nicht symmetrisch substituierten Olefinen zu isomeren Produkten. Aus der US-A 3,679,759 ist bekannt, die Ausbeute dadurch zu erhöhen, daß die Reaktion unter Druck durchgeführt wird. Dies erfordert jedoch aufwendige Druckvorrichtungen.

Lediglich im Falle hochreaktiver konjugierter Diene und Enine wurden bislang rasche Umsetzungen beobachtet, die Ausgangspunkt präparativer Verfahren sind [W. H. Carothers et al., J. Am. Chem. Soc. 55, 787 (1933)].

Aufgabe der Erfindung ist es also, die Herstellung von Alkylbromiden aus wäßriger Bromwasserstoffsäure und Olefinen zu verbessern. Insbesondere sollen das Verfahren vereinfacht und die Kosten gesenkt werden.

Diese Aufgabe wird dadurch gelöst, daß ein Verfahren zur Herstellung von Alkylbromiden aus Mischungen, enthaltend wäßrige Bromwasserstoffsäure und Olefine, bereitgestellt wird, bei dem während der Umsetzung die vorhandene Bromwasserstoffsäure aufkonzentriert wird.

Diese Aufkonzentration erfolgt in vorteilhafter Weise durch Abdestillieren von Wasser während der Umsetzung, insbesondere dadurch, daß das Wasser in Form eines Azeotrops mit einer in der Reaktionsmischung vorhandenen organischen Komponente abgetrennt wird, die anschließend der Reaktionsmischung wieder zugeführt wird.

Weitere bevorzugte Ausführungsformen der Erfindung sind Gegenstand der Unteransprüche.

Dadurch, daß erfindungsgemäß die vorhandene Bromwasserstoffsäure aufkonzentriert wird, kann ein günstigeres Verhältnis der Einsatzstoffe gewählt werden, so daß auch die in technischem Maßstab leicht zugängliche, kostengünstige wäßrige Bromwasserstoffsäure zur Synthese von Alkylbromiden ausgehend von Olefinen eingesetzt werden kann. Ein weiterer Vorteil liegt darin, daß die zugesetzte Säure bis zum Ende der Umsetzung vollständig aufgebraucht und das verbleibende Wasser - u.U. mit Spuren von HBr - abdestilliert wird. Dadurch ist eine anschließende einfache Aufarbeitung des gebildeten Alkylbromids nach der Umsetzung durch direkt darauffolgende Destillation ohne dazwischengeschaltete Phasenseparations- und/oder Extraktionsschritte möglich.

Erfindungsgemäß kann das Aufkonzentrieren der Bromwasserstoffsäure durch Abdestillieren des Wassers allein erfolgen. Es ist jedoch bevorzugt, das Wasser in Form eines Azeotrops mit einer in der Reaktionsmischung vorhandenen organischen Komponente abzutrennen. Die mit dem Wasser ein Azeotrop bildende Komponente ist bevorzugt Cyclopentan, Cyclohexan, Benzol, Toluol. Sie kann in vorteilhafter Weise durch Phasentrennung im Destillat kontinuierlich abgetrennt und dem Reaktionsgemisch wieder zugeführt werden. Ganz besonders vorteilhaft ist es, wenn es sich bei der mit dem Wasser ein Azeotrop bildenden Komponente um das Alkylbromid, d.h. das Reaktionsprodukt handelt. In diesem Falle erhält man nach Phasentrennung in Destillat ein bereits weitgehend vorgereinigtes Produkt.

Die Zugabe der Ausgangskomponenten zu der Reaktionsmischung kann so erfolgen, daß sowohl das Olefin zur Bromwasserstoffsäure als auch umgekehrt die Bromwasserstoffsäure zum Olefin hinzugefügt wird. Bei Verwendung von einem oder mehreren Lösungsmitteln können diese wahlweise ebenfalls vorgelegt oder aber den übrigen Reaktanten zugefügt werden.

Während der Umsetzung wird die Mischung in vorteilhafter Weise, bevorzugt durch Verwendung einer mechanischen Rührvorrichtung, durchmischt.

Die Reaktion erfolgt bei einem Absolutdruck zwischen 0,001 und 1000, bevorzugt zwischen 0,1 und 10 bar. Besonders bevorzugt ist eine Durchführung der Reaktion bei Normaldruck. Die Reaktionstemperatur liegt zwischen -50 bis 400°C, bevorzugt zwischen -20 und 150°C. Besonders vorteilhaft ist eine Reaktionstemperatur, die zwischen 0 °C und dem Siedepunkt der jeweiligen Mischung bei Normaldruck liegt, da sich dann die Verfahrensführung besonders einfach gestaltet.

Die Bromwasserstoffsäure wird als wäßrige Lösung mit einer Konzentration im Bereich zwischen 0,1 und 95 Gew.-%, insbesondere 1 bis 80 Gew.-%, bevorzugt zwischen 40 und 65 Gew.-% eingesetzt.

Das Olefin oder die Mischung der umzusetzenden Olefine kann mit der wäßrigen Bromwasserstoffsäure allein oder in Gegenwart eines oder mehrerer Lösungsmittel umgesetzt werden. Diese Lösungmittel können nicht vermischbar oder aber auch mischbar sein; bevorzugt ist jedoch eine Durchführung der Reaktion ohne Lösungsmittel oder in Gegenwart eines Alkans bzw. Alkangemischs wie insbesondere Cyclopentan, Cyclohexan oder Gemische isomerer (Cyclo-)pentane oder (Cyclo-)hexane.

Das bei der Reaktion eingesetzte Olefin bzw Olefingemisch besitzt die allgemeine Formel R¹(R²)C=C(R³)R⁴, wobei R¹, R², R³ und/oder R⁴ jeweils Wasserstoff, Deuterium oder ein - wahlweise mit funktionellen Gruppen substituierter - C₁-C₂₀₀-Alkyl-, -C₃-C₂₀₀-Cycloalkyl-, C₂-C₂₀₀-Alkenyl-, C₃-C₂₀₀-Cycloalkenyl- oder C₆-C₂₀₀-Alylrest ist. Die erwähnte funktionelle Gruppe ist unter den Reaktionsbedingungen chemisch inert. Vorzugsweise handelt es sich dabei um einen Aryl-, einen Halogenid- oder einen Nitrorest. Außerdem können R¹, R², R³ und/oder R⁴ auch Teile gemeinsamer Ringe sein, insbesondere von C₁-C₂₀₀-Alkylen-, C₃-C₂₀₀-Cycloalkylen-, C₂-C₂₀₀-Alkenylen-, C₃-C₂₀₀-Cycloalkenylen-, oder C₆-C₂₀₀-Arylengruppen.

Bevorzugt sind C₁-C₅-Alkylengruppen, insbesondere C₃-C₄-Alkylenreste. Ferner können auch R¹ unter Wegfall von R² sowie R³ unter Wegfall von R⁴ doppelt gebunden sein, d.h. in diesem Falle handelt es sich um C₁-C₂₀₀-Alkyliden, C₃-C₂₀₀-Cycloalkyliden-, C₂-C₂₀₀-Alkenyliden-, C₃-C₂₀₀-Cycloalkenyliden oder C₆-C₂₀₀-Arylidengruppen.

Die Reaktion kann mit dem reinen Olefin oder aber auch mit einer Mischung verschiedener Olefine durchgeführt werden, wie sie beispielsweise bei der Dampfspaltung längerkettiger Kohlenwasserstoffe, bei Eliminierungsreaktionen von beispielsweise Alkoholgemischen, bei Pyrolysen von Kohlenwasserstoffen, bei Dehydrierung von Alkanen oder Hydrierungen von Alkinen, bei der Wittig- oder der Wittig-Homer-Reaktion entstehen können.

Die Umsetzung kann ohne oder in Gegenwart eines geeigneten Katalysators durchgeführt werden. Als Katalysatoren können beliebige Elemente M oder Verbindungen, beispielsweise Salze, Koordinationsverbindungen, Komplexverbindungen, der allgemeinen Formel M_{W}LₓA_{y}(H₂O)_{z} oder Mischungen sowie Legierungen derselben verwendet werden, wobei
- M ein oder mehrere Elemente der Gruppen IA, IIA, IIIB, IVB, VB, VIB, VIIB, VIII, IB, IIB, IIIA oder IVA des Periodensystems sowie der Lanthaniden, bevorzugt ein oder mehrere Elemente der Gruppen IIA, VB, VIB, VIIB, VIII, IB, IIB und IIIA des Periodensystems, besonders bevorzugt Eisen, Zink oder Aluminium,
- L ein- oder mehrzähniger Ligand oder eine Kombination verschiedener ein- oder mehrzähniger Liganden wie beispielsweise Carboxylate, Cyanid, Halogenid, Amin, Amid, Phosphin, Phosphonat, Nitrat, Nitrit, Alkoholat, Sulfid, Thiolat, Sulfonat, Olefin, Aryl, Nitril, Imin oder Carbonyl,
- A ein oder mehrere Elemente oder eine oder mehrere Verbindungen von Elementen der Gruppen VB, VIB, IIIA, IVA, VA, VIA oder VIIA des Periodensystems, bevorzugt ein Borat, Carboxylat, Carbonat, Cyanid, Amid, Nitrat, Nitrit, Phosphat, Phosphonat, Polyphosphat, Heteropolyphosphat des Molybdäns oder des Wolframs, Oxid, Hydroxid, Alkoholat, Sulfid, Sulfat, Thioat, Sulfinat, Sulfonat, Selenid, Halogenid, Halogenat oder Perhalogenat, besonders bevorzugt Bromid oder Hydroxid,
- w mindestens 1,
- x größer oder gleich Null,
- y mindestens 1,
- z größer oder gleich Null sind.

Der Katalysator kann in reiner Form, in Lösung oder auch auf einem Trägermaterial eingesetzt werden. Als Träger können organische Harze wie beispielsweise Ionentauscherharze, Molsiebe wie zum Beispiel Montmorillonite, Kaolinite und Bentonite oder auch Zeolithe, poröse Materialien aus Kohlenstoff wie beispielsweise Aktivkohle sowie Oxide, Sulfate, Sulfide, Hydroxide, Halogenide, Phosphate oder Pyrophosphate von Elementen der Gruppen IA, IIA, IIIB, IVB, VIB, VIIB, VIII, IB, IIB, IIIA oder IVA des Periodensystems sowie der Lanthaniden eingesetzt werden. Bevorzugt ist der Einsatz in reiner Form. Der reine oder derart geträgerte Katalysator kann sowohl als Pulver, als Splitt als auch als Formkörper wie beispielsweise in Strängen verwendet werden.

Das Molverhältnis Olefin:HBr beträgt in der Regel 0,001:1 bis 1000:1, bevorzugt 0,1:1 bis 10:1 und besonders bevorzugt 0,2:1 bis 5:1.

Das Molverhältnis des eingesetzten Katalysators zum eingesetzten Olefin kann zwischen 0,001 und 1000, bevorzugt zwischen 0,01 und 10 und besonders bevorzugt zwischen 0,1 und 1 Mol% gewählt werden.

Die sich an die Umsetzung anschließende Aufarbeitung kann in unterschiedlicher, in ihrer Art allgemein bekannter Art und Weise erfolgen. Bevorzugt jedoch wird die Reaktionsmischung ohne weitere vorgeschaltete Aufarbeitungsschritte direkt durch Destillation des Produkts aufgearbeitet.

Nachfolgend werden Beispiele für das erfindungsgemäße Verfahren gegeben:

### Beispiele

### Beispiel 1

136 g Cyclopenten, 180 g Cyclopentan, 310 g 47%ige wäßrige HBr und 2,7 g Zinkbromid wurden in einer Rührapparatur mit Wasserabscheider unter Rückfluß zum Sieden erhitzt, bis sich keine wäßrige Phase im Wasserabscheider mehr bildete. Der Umsatz an Cyclopenten lag bei 90 % der Theorie (GC, FID-Flächen), die Selektivität für Cyclopentylbromid jeweils bei 99%. Die Aufarbeitung erfolgte durch Destillation unter vermindertem Druck. Auf diese Weise erhielt man beispielsweise aus dem angegebenen Ansatz ca. 220 g reines Cyclopentylbromid.

### Beispiel 2

Es wurde analog zu Beispiel 1 verfahren, statt 2,7 g Zinkbromid wurden 4,8 g Eisen(III)sulfat verwendet. Der Cyclopenten-Umsatz lag nach beendeter Wasserabtrennung bei 98 % der Theorie (GC, FID-Flächen), die Selektivität bei 99%.

### Beispiel 3

Es wurde analog zu Beispiel 1 verfahren, statt 2,7 g Zinkbromid wurden jedoch 4,1 g Aluminiumsulfat verwendet. Der Cyclopenten-Umsatz nach beendeter Wasserabscheidung lag bei 90 % der Theorie (GC, FID-Flächen), die Selektivität bei 99 %.

### Beispiel 4

Es wurde analog Beispiel 1 verfahren, statt 136 g Cyclopenten und 180 g Cyclopentan wurden jedoch 164 g Cyclohexen und 168 g Cyclohexan verwendet. Der Cyclohexenumsatz lag nach beendeter Wasserabtrennung bei 98 % der Theorie (GC, FID-Flachen), die Selektivität bei 99%. Auf diese Weise erhielt man aus dem angegebenen Ansatz 250 g reines Cyclohexylbromid.

## Patentansprüche

1. Verfahren zur Herstellung von Alkylbromiden aus Mischungen, enthaltend wäßrige Bromwasserstoffsäure und Olefine, dadurch gekennzeichnet, daß während der Umsetzung die vorhandene wäßrige Bromwasserstoffsäure aufkonzentriert wird.

2. Verfahren nach Anspruch 1, dadurch gekennzeichnet, daß das Aufkonzentrieren durch Abdestillieren von Wasser oder eines Azeotrops von Wasser mit einer organischen Komponente erfolgt.

3. Verfahren nach einem der vorhergehenden Ansprüche, dadurch gekennzeichnet, daß die Reaktion bei einem Absolutdruck zwischen 0,1 und 10 bar, bevorzugt bei Normaldruck durchgeführt wird.

4. Verfahren nach einem der vorhergehenden Ansprüche, dadurch gekennzeichnet, daß die Reaktionstemperatur zwischen -20 und 150°C, bevorzugt zwischen 0°C und dem Siedepunkt der jeweiligen Mischung bei Normaldruck liegt.

5. Verfahren nach einem der vorhergehenden Ansprüche, dadurch gekennzeichnet, daß die wäßrige Bromwasserstoffsäure in einer Konzentration von 1 bis 80 Gew.-%, bevorzugt 40 bis 65 Gew.-% eingesetzt wird.

6. Verfahren nach einem der vorhergehenden Ansprüche, dadurch gekennzeichnet, daß ein Olefin der allgemeinen Formel R¹(R²)C=C(R³)R⁴ eingesetzt wird, wobei R¹, R², R³ oder R⁴ jeweils unabhängig voneinander Wasserstoff, Deuterium oder ein wahlweise mit funktionellen Gruppen, insbesondere substituierter C₁-C₂₀₀-Alkyl-, C₃-C₂₀₀-Cycloalkyl-, C₂-C₂₀₀-Alkenyl-, C₃-C₂₀₀-Cycloalkenyl- oder C₆-C₂₀₀-Arylrest sind, durch eine Aryl-, eine Halogenid- oder eine Nitrogruppe oder daß R¹ unter Wegfall von R² sowie R³ unter Wegfall von R⁴ als C₁-C₂₀₀-Alkyliden, C₃-C₂₀₀-Cycloalkyliden, C₂-C₂₀₀-Alkenyliden, C₃-C₂₀₀-Cycloalkenyliden oder C₆-C₂₀₀-Aryliden doppelt gebunden sind.

7. Verfahren nach einem der vorhergehenden Ansprüche, dadurch gekennzeichnet, daß als Olefin Cyclopenten rein oder in Mischung mit C₁-C₁₀-Olefinen verwendet wird.

8. Verfahren nach einem der vorherigen Ansprüche, dadurch gekennzeichnet, daß als Lösungsmittel bei der Umsetzung ein Gemisch von Kohlenwasserstoffen verwendet wird, das zu über 50% aus C₁-C₁₀-Kohlenwasserstoffen besteht und mit Wasser unter den Reaktionsbedingungen ein Azeotrop bildet.

9. Verfahren nach Anspruch 8, dadurch gekennzeichnet, daß das Molverhältnis von Cyclopenten zu HBr zwischen 2:1 und 0,2:1 liegt.

10. Verfahren nach einem der vorhergehenden Ansprüche, dadurch gekennzeichnet, daß in der Reaktionsmischung zusätzlich ein Katalysator vorhanden ist, der eine oder mehrere Verbindungen gemäß der allgemeinen Formel M_{W}LₓA_{y}(H₂O)_{z} umfaßt, wobei
- M eines oder mehrere Elemente der Gruppen IA, IIA, IIIB, IVB, VB, VIB, VIIB, VIII, IB, IIB, IIIA oder IVA des Periodensystems oder der Lanthanide bevorzugt Eisen, Zink, und/oder Aluminium ist,
- L ein ein- oder mehrzähniger Ligand oder eine Kombination verschiedener ein- oder mehrzähniger Liganden, insbesondere von Carboxylaten, Cyaniden, Halogeniden, Aminen, Amiden, Phosphinen, Phosphonaten, Nitraten, Nitriten, Alkoholaten, Sulfiden, Thiolaten, Sulfonaten, Olefinen, Arylen, Nitrilen, Iminen oder Carbonyl ist,
- A ein oder mehrere Elemente oder Verbindungen von Elementen der Gruppen VB, VIB, IIIA, IVA, VA, VIA oder VIIA des Periodensystems, insbesondere ein oder mehrere Verbindungen umfaßt, ausgewählt aus der Gruppe Borat, Carboxylat, Carbonat, Cyanid, Amid, Nitrat, Nitrit, Phosphat, Phosphonat, Polyphosphat, Heteropolyphosphat des Molybdäns oder Wolframs, Oxid, Hydroxid, Alkoholat, Sulfid, Sulfat, Thiolat, Sulfonat, Sulfinat, Selenid, Halogenid, Halogenat und Perhalogenat, bevorzugt ausgewählt aus Bromid und Hydroxid,
und
- w mindestens 1,
- x größer oder gleich Null,
- y mindestens 1 und
- z größer oder gleich Null sind.
